# EUROPEAN PATENT SPECIFICATION

(11) **EP 1 987 346 B1**
(45) Date of publication and mention of the grant of the patent: **11.08.2010**
(21) Application number: 07705132.4
(22) Date of filing: 06.02.2007
(51) Int. Cl.: G01N 21/03, G01N 21/35, G01J 3/42, G01N 33/00

(54) **DOME GAS SENSOR**
KUPPELGASSENSOR
DÉTECTEUR DE GAZ EN FORME DE DÔME

(30) Priority: 06.02.2006 GB 0602320
(43) Date of publication of application: 05.11.2008
(73) Proprietor: GAS SENSING SOLUTIONS LIMITED, Westfield North Courtyard Cumbernauld Glasgow City G68 9HQ (GB)
(72) Inventor: SMITH, Michael, J., Carmarthenshire SA32 8LX (GB)
(74) Representative: Hindle, Alistair Andrew
(86) International application number: PCT/GB2007/000401
(87) International publication number: WO 2007/091043

(56) References cited:
- EP-A- 0 704 691
- WO-A-00/07411
- DE-A1- 10 200 797
- DE-A1- 10 200 908
- GB-A- 2 391 310

## Description

The present invention relates to gas sensing, in particular gas sensors such as non-dispersive infrared (NDIR) gas sensors having a radiation source, radiation detector and a reflector arranged to reflect radiation from the radiation source to the radiation detector.

In the field of gas sensing, there is a requirement for small, low cost gas sensors that can operate over a wide range of environmental conditions. This is driven by legislation directed to increasing safety and reducing emissions in a variety of applications. For example, in the automotive industry, sensing of the presence of automotive exhaust gases and CO₂ in vehicle cabins and engine management systems are applications where a small form factor as well as low cost and efficiency are desirable. The need for detection of C0₂ in vehicle cabins comes from the move towards C0₂ refrigerant based air conditioning systems away from the use of more environmentally harmful Fluorocarbon based refrigerants such as P134a. By providing C0₂ based air conditioning systems, automotive manufacturers will be able to avoid emission penalties applied to the disposal and recycling of hydrofluorocarbons. However, conventional gas sensors suitable for C0₂ and C0 gas sensing are too large and too expensive for use in such automotive applications. Furthermore, in such applications, the gas sensor is required to operate over a wide range of temperatures.

Like the automotive industry, industrial heating, ventilation and air conditioning (HVAC) systems based on C0₂ refrigerants require low cost C0₂ gas sensors that operate in a variety of environments. The safety products that detect combustion or solvent gas leaks in many applications, from gas welding through automatic production processes to solvent cleaners, also require low-cost, efficient gas sensors.

In the domestic heating field, gas sensors are used to provide safety from carbon monoxide poisoning. Furthermore, combustion gas sensing provides safety from an explosion risk.

In gas sensing, infrared gas sensors have advantages compared with other technologies, including long lifetime and resistance to poisoning. However many infrared detectors use thermal components such as incandescent sources (e.g. bulbs) and pyro-electric or thermopile detectors, which themselves have several disadvantages. For example they may have a slow response or a limited wavelength range and may require explosion-proof housing to prevent the bulb acting as an ignition source.

Replacing the incandescent sources and thermal detectors with high performance LEDs (Light Emitting Diodes) and photodiodes offers advantages including low power, fast response and intrinsic safety, for a greater range of gases.

Gas sensors may be made using a LED and a photodiode that are manufactured at matched frequencies such that they have stable and very narrow coincident optical bandwidths in operation.

In an NDIR gas sensor, light is emitted from a light source, passed through a gas and then measured by a light detector. For efficient detection of a gas, it is important to have a large interaction between the light and the gas and this is influenced by the length and volume of the interacting optical path, and the transport of the gas into and out of the interacting optical path. The problems with simply arranging a detector in front of an emitter are that when the light diverges from the source, only a small proportion of the light is incident upon the detector and the optical path length is merely the distance around the emitter and the detector. Therefore, there is a relatively small length and volume for the gas to interact with the light. Known approaches to improve this arrangement are to coat the internal walls of the sensor housing with reflective material and to fold the optical path by the use of mirrors. However, though the folded linear path the latter approach can increase the optical path length, it retains the problem that the path only sweeps out part of the available volume between the light source and the light detector, even using curved mirrors. Thus with a folded optical path, only a fraction of the available volume of the sensor housing is used for the interacting optical path. Also, a multiply folded optical path, for example in a zig-zag shape, requires multiple reflectors that need to be well aligned.

GB2391310 A discloses a gas sensor using the principle of infrared (IR) absorption in which the optical source emits IR radiation into a gas cell. The IR radiation is reflected and focussed such that said IR radiation makes multiple passes within the gas cell before reaching the detector. Further aspects disclosed include multiple focussing reflectors, diffraction gratings and flameproof housing for all or part of the sensor.

WO0007411 A1 discloses a blackbody radiation device that includes a planar filament emission element and a planar detector for respectively producing and detecting radiation; a reflector; a window; an electrical control; and a data output element.

EP0704691 A2 discloses a spectrometric sensor that has a single moulded block forming a microstructure body with a base plate, a mirror grid, an input slot for reception of a poly-frequency IR beam and an output slot for a mono-frequency IR beam. The sample gas is contained in the free space between the entry and exit slots and the mirror grid, enclosed by cover plates at either end with gas inlet and exit openings. The mirror grid is provided by a concave reflection grid, with the mirror grid and the entry and exit slots for the IR radiation positioned on a Rowland circle, or along a curve in the vicinity of the Rowland circle.

DE10200797 A1 discloses an infra-red (IR) absorption gas sensor for determining the concentration of IR active gases in mixtures that comprises a radiation source and a detector which are displaced with respect to the optical axis. The mirror used for the image has a spherical surface, and the mirror comprises plastic with an IR reflective coating.

DE10200908 A1 discloses an infrared gas sensor that comprises an infrared radiator, an infrared detector and a wavelength-selective element. The optical axes of the radiator and detector are in the same direction. Infrared radiation is deviated via an imaging lens by 90 degrees as a parallel light bundle in the direction of the optical axis of the detector, and focussed onto the detector via a further imaging lens over a path. The imaging lens consists of a spherical or parabolic surface with a focal point containing the infrared radiator. The sensor has a holder and a cap both made from plastic. All surfaces in contact with the radiation and the lenses are coated with an infrared-reflecting layer.

It is an object of the present invention to provide a compact, high-efficiency gas sensor in which the dome functions to reflect radiation from the emitter through a single reflection to the detector irrespective of the radiation exit angle from the emitter.

According to the present invention, there is provided a gas sensor comprising:
a radiation source;
a radiation detector; and
a reflecting means arranged to reflect radiation from the radiation source to the radiation detector along an optical path,
wherein the radiation source and the radiation detector are disposed side by side and
the reflecting means is arranged to reflect radiation divergent from the radiation source and to concentrate the reflected radiation onto the radiation detector, and wherein the reflecting means comprises a surface comprising a plurality of sub surfaces, each defined by an arc with a radius and a centre point, the sub surfaces being swept out by rotation of the arcs around an axis, and each sub surface being tangent to an adjacent sub surface and having a different radius and different centre point from the adjacent sub surface.

Preferably, the axis is in line with the radiation source and the radiation detector.

Preferably, the arc length tends to zero.

Preferably, the sub surfaces are semi-toroidal.

Preferably, the surface is configured to reflect radiation from the radiation source to a corresponding location on the radiation detector, irrespective of the radiation exit angle from the radiation source.

Preferably, the surface is configured to reflect radiation leaving the centre of the radiation source to the centre of the radiation detector, radiation leaving the outer side of the radiation source to the outer side of the radiation detector, and radiation leaving the inner side of the radiation source to the inner side of the radiation detector.

Preferably, the surface is configured to reflect radiation such that the length of the optical path is on average equal for each sub surface.

Preferably, the reflecting means comprises a reflective surface of a housing.

Preferably, the housing has at least one aperture for permitting the transport of gas in and out of the gas sensor.

Preferably, the radiation source and radiation detector are mounted on a common substrate configured to locate the radiation source and radiation detector in relation to the housing.

Preferably, the substrate is configured as an elongate member extending along a diameter of the housing.

Preferably, the elongate member is adjustable so as to optimise the location of a reflected radiation pool on the radiation detector.

Preferably, the elongate member is adjustable by sliding of pins.

Preferably, the pins are electrical leads.

Preferably, the adjustable elongate member is lockable with respect to the reflecting means.

Preferably, the adjustable elongate member is lockable by gluing the pins to the reflecting means.

Preferably, the adjustable elongate member is lockable by soldering the pins.

Preferably, the reflecting means is arranged such that the optical path is defined at least in part by a cavity extending around the radiation source and radiation detector.

Preferably, the cavity is bounded by a plane parallel to surfaces of the radiation source and the radiation detector.

Preferably, the surface is configured such that radiation originating from a point on the radiation source is unfocussed as it converges on the radiation detector.

The present invention will now be described by way of example only with reference to the figures in which:
Figure 1 illustrates in schematic form a cross section of a first embodiment of a gas sensor;
Figure 2 illustrates in schematic form a cross section of the radiation source and radiation detector assembly;
Figure 3 illustrates in schematic form a cross section of a second embodiment of a gas sensor;
Figure 4 illustrates in schematic form a perspective view of the second embodiment of a gas sensor;
Figure 5 illustrates in schematic form a half cross section of the dome reflector;
Figure 6 illustrates in schematic form rays of light being reflected between the centres and outer sides of the radiation source and radiation detector;
Figure 7 illustrates in schematic form rays of light being reflected between the centres and inner sides of the radiation source and radiation detector; and
Figure 8 illustrates in schematic form a cross section of an embodiment of the gas sensor having an adjustable bridge.

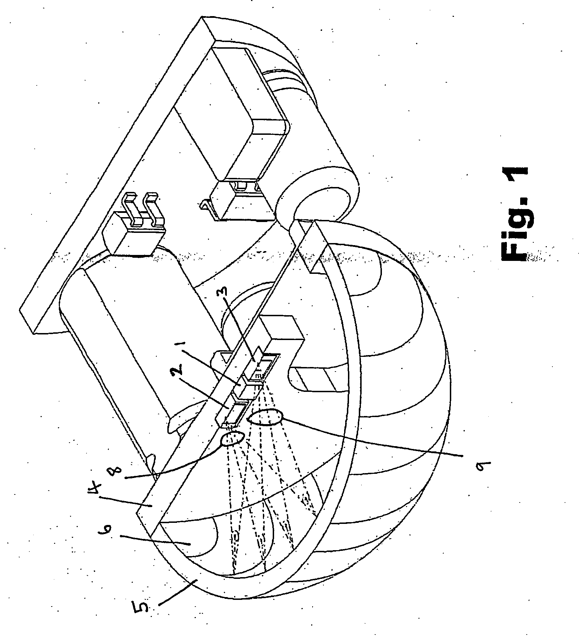

With reference to figure 1, a partial cross section of a gas sensor in accordance with a first embodiment of the present invention is shown. The gas sensor has a screen 1 in between a LED radiation source 2 and a photodiode radiation detector 3 on a substrate 4 mounted within the gas sensor. The LED and photodiode are thereofore side-by-side, with the screen in line and in between them. Only half of a housing 5 and the radiation path is shown in figure 1. The housing has radial symmetry centred on the LED/screen/photodiode assembly. The inner surface 6 of the housing is reflective. This may be achieved by applying a reflective coating to a moulded plastic housing. Light rays 8 that diverge from the LED are reflected from the inner surface of the housing. The housing is shaped such that the light emitted by the LED is reflected through the cavity extending around the LED and photodiode and the reflected light rays 9 are concentrated onto the photodiode. The cavity is bounded by the plane parallel to the main emitting and absorbing surfaces of the LED and photodiode respectively. The rays reflected from surface 6 may or may not be focused. The curved shape of the housing is arranged to provide an even, broad spread of the light throughout the cavity. The surface may be hemispherical or semi-ellipsoidal. The even spread of light through the cavity is generally characterised by avoidance of focus where light rays originating from a point on the source do not converge on a particular focal point, but none the less converge on the photodiode. The side-by-side geometry has the advantage of allowing a small housing with a maximum spread of the interacting light path throughout the available volume of the housing. This provides good optical absorption efficiency and minimises the risk of saturation of the gas's interaction with the light, all in a compact housing. The compact optical design enables the gas sensor to fit within a 20mm diameter and 17mm long form factor. These features improve the gas sensor's sensitivity for gas sensing and response and the compact size makes it suitable for use in a wide range of space sensitive applications, where large housings are not acceptable.

The LED has a narrow emission bandwidth, therefore using a LED and photodiode the narrow optical bandwidth required for gas sensing may be achieved without optical filters as are required for incandescent and other sources. However, the radiation source may be a LED that uses an optical bandpass filter to trim the optical emission profile but remove all other light frequencies that may cause error in the gas sensing process. Such an optical bandpass filter may remove no more than 25% of the emitted light form the LED, whereas in the prior art case of an incandescent source the vast majority of radiated light would be removed by the filter. Therefore, the LED radiates a precise and narrow bandwidth which is not post or pre optically filtered other than by simple bandwidth trimming.

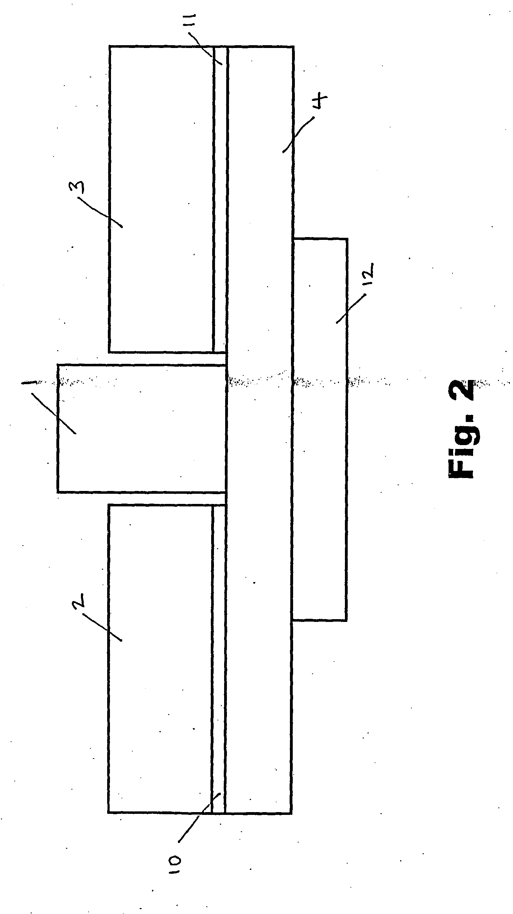

Figure 2 shows a cross section of the radiation source, screen and radiation detector is shown. With reference to figure 2, the LED radiation source 2 and the photodiode radiation detector 3 are side-by-side mounted on an interconnecting substrate 4, with the screen 1 in between. The screen may be formed as part of the substrate and the LED and/or photodiode may abut the screen. The screen may be reflecting. The surfaces of the LED and/or photodiode facing the screen may be reflecting. Alternatively the screen may be a reflective coating on one or more of the surfaces of the LED or photodiode facing each other.

Both the radiation source and detector in this embodiment are based on the narrow band gap III-V material indium aluminium antimonide (In₍₁₋ₓ₎AlₓSb), grown on a gallium arsenide (GaAs) substrate, the band gap of which can be tuned to a very narrow width to provide light emission and detection that is specific to carbon dioxide (C0₂) and carbon monoxide (CO gases) or other selected gases without the use of expensive optical filters and complicated differentiating circuitry. The LED and photodiode may be fabricated from the same semiconducting substrate. The LED and photodiode may also be fabricated from very similar substrates varying only by their epilayer thicknesses, which maybe tuned to enhance the performance of light emission in the case of the LED or collection in the case of the photodiode. In other embodiments, the radiation source and radiation detector may comprise one or more discrete LED or photodiode elements respectively.

The invention is not limited to this type of radiation source and radiation detector. For example, cadmium mercury telluride compounds are useful with ultraviolet frequencies. Although solid state radiation sources and detectors are convenient for miniaturised application, the present invention may also be implemented using incandescent sources and pyro-electric or thermopile detectors.

The interconnecting substrate 4 and/or screen is thermally conductive and provides thermal communication between the LED and the photodiode. The thermal communication allows the transfer of heat from the LED to the photodiode. This provides the advantage of reducing the temperature difference between the LED and photodiode, thereby simplifying the compensation of any temperature dependent effects on the operation of the LED and/or photodiode. This approach is in contrast to most common electrical applications of conductive layers where the heat is transferred away from the semiconductors. The heating effect may be used to keep the photodiode at an elevated temperature when compared to its surroundings, thus keeping it on the positive side of the dew point of the ambient gas, therefore reducing the risk of condensation forming on the photodiode.

The substrate may have integrated in it or mounted on it a temperature control means 12 such as a heater or cooler (Peltier device or similar) that can be controlled and powered to affect the temperature of the LED and photodiode simultaneously.

Temperature detection maybe achieved by use of an additional device (not shown), which may be in or on the substrate, such as a thermistor, or may be detected by measuring the characteristics of either the emitter or detector. For example the forward voltage of the LED will vary with temperature.

The substrate provides a structural mounting for the LED and photodiode within the gas sensor. The substrate may be shaped to aid in locating the radiation source or detector for mounting on the substrate. The substrate may also provide mechanical features that serve to precisely locate the optical pair within the optical housing avoiding the need for adjustment or setting during the assembly process.

The LED and photodiode are each provided with optically reflective layers 10, 11 on their surfaces. These reflective layers may be included on one or other of the LED and photodiode, or not at all. The reflective layers may be part of the substrate or applied as a coating to the back and/or sides of the LED and/or photodiode. The optical reflection improves the efficiency of both the LED and the photodiode. A proportion of the light generated or detected can pass straight through either device without being absorbed, however the incorporation of the reflective layers functions to return the light back through the LED improving emission efficiency, or similarly in the case of the photodiode, it can significantly increase the absorption by reducing loss of light out of the back or sides of the photodiode.

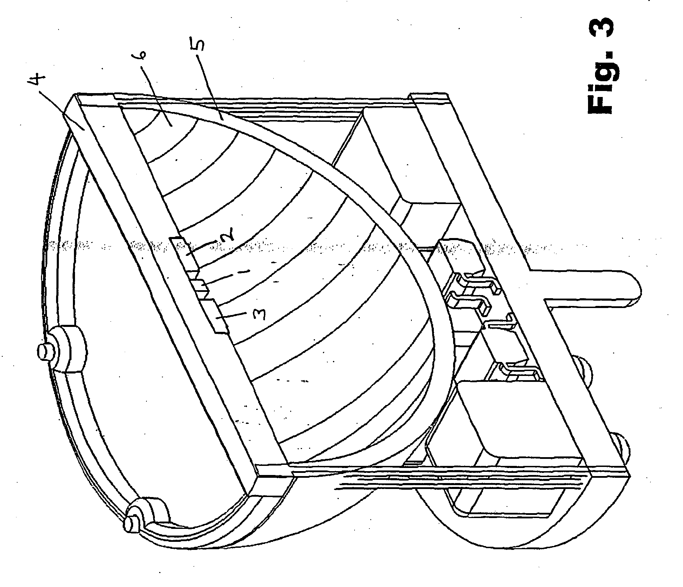

With reference to figure 3, a cross section of a second embodiment of a gas sensor is shown. The elements are numbered as in figure 1. In comparison with the first embodiment illustrated in figure 1, the domed reflector has been inverted. In this embodiment the substrate is an elongate printed circuit board extending along a diameter of the domed reflecting housing, which has radial symmetry. This substrate allows the mounting of further components (not shown). These components can include a temperature sensor (12 in figure 2) that because of the side-by-side mounting of the LED and photodiode allows the measurement simultaneously by one sensor of the temperature of both the LED and the photodiode. This has the advantage of reducing the component count. Another component or set of components that can be incorporated adjacent to the LED and photodiode are signal processing elements, including a preamplifier. For example, the preamplifier may be mounted on the substrate next to the LED/photodiode pair with the remaining electronics and processing components being located at the next available position on the substrate away from the LED/photodiode pair. In that case there is an advantage that the preamplifier and processor are located adjacent to both the LED and the photodiode. Any electrical modulation signals can be transmitted to the LED with minimised noise pickup. Furthermore, the same components can detect the signal from the photodiode, which may be in the nA range. These low level signals are similarly sensitive to noise pickup effects and the location of the processing elements adjacent to the detector reduces the effect of such noise pickup. There is a further advantage to having the signal amplification and processing components within a gas sensor housing, which is the shielding afforded by the metalised housing. The apertures in the housing may reduce such shielding, but the circuits containing the components may be designed to balance with any antenna affect of the housing in order to achieve a zero biased system.

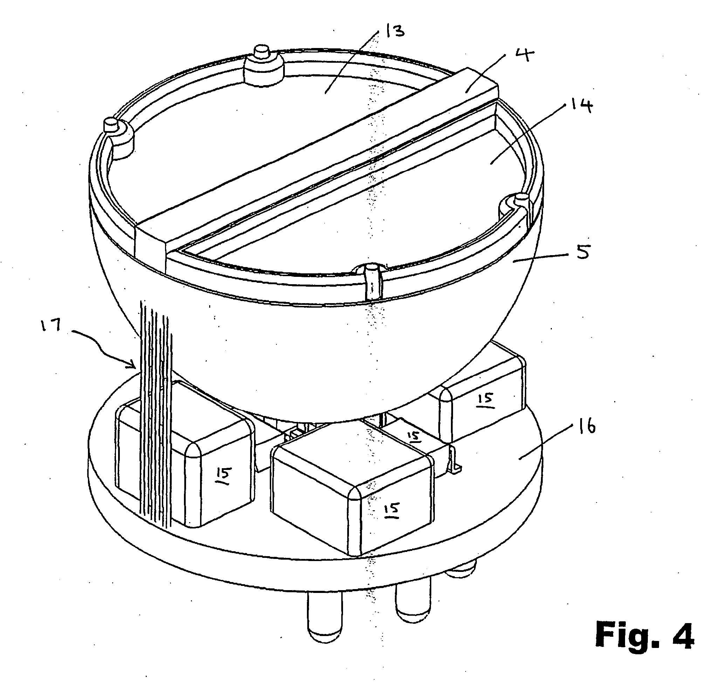

With reference to figure 4, a perspective view of the second embodiment of a gas sensor is shown. Two gas filters 13, 14 are shown with the very narrow printed circuit board (PCB) 4 being located in the middle of them. As an alternative to the signal processing and temperature control components 15 being located on the emitter/collector PCB as discussed above, they are placed on a second PCB 16. Having the preamplifier thus separated from the LED/photodiode pair has been enabled by the use of a centre tap connection in the centre of an array of discrete photodiode elements that make up the radiation detector connected along with the other two LED terminations by pins 17 to either:
a) two independent transimpedance amplifiers and the outputs differentially amplified to combine the signals and cancel out any common noise; or
b) a differential transimpedance amplifier.

A tubular external housing (not shown) may be placed around the assembly, which forms a Faraday cage between the metalised reflector, the external tube and a shielding layer built into the second PCB, so improving the electrical isolation of the components, as well as being a structural support.

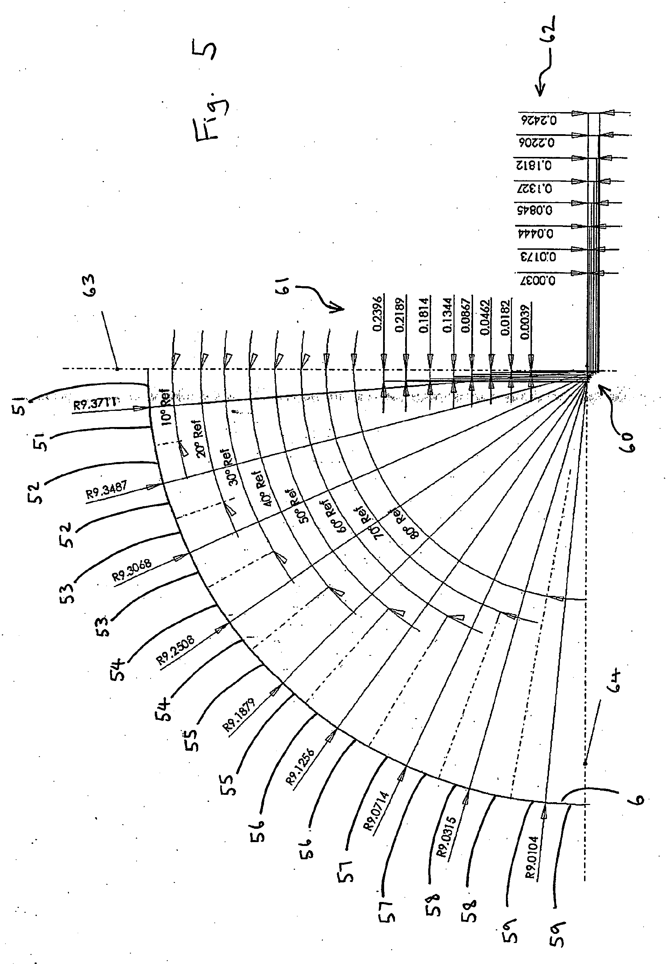

Figure 5 shows a cross section of half of the internal surface 6 of the dome in another embodiment of the present invention. The dome has an internal surface comprising a plurality of sub surfaces 51 to 59, shown in section as arcs, each defined by a radius R9.3711 to R9.0104 respectively and a centre point 60, each sub surface being tangent to an adjacent sub surface and having a different radius and different centre location from the adjacent subsurface. The offsets of the centre points 61, 62 from the perpendicular datum lines 63 and 64 respectively are also shown. The emitter and detector lie on the datum line 64. The surface is defined by the arcs labelled 51 to 59 (and their reflection about datum line 63) being swept out by rotation by 180 degrees around datum line 64. Another embodiment may have the arc length tending to zero, thus giving a continuously varying curve from datum line 63 to datum line 64.

The internal sub surfaces of this embodiment are semi-toroidal. The internal surface therefore does not form a focused reflector.

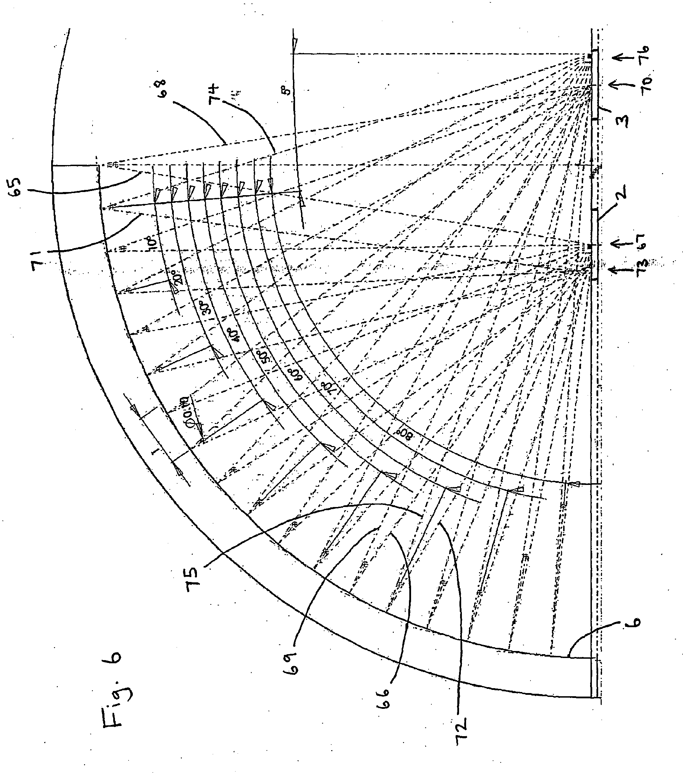

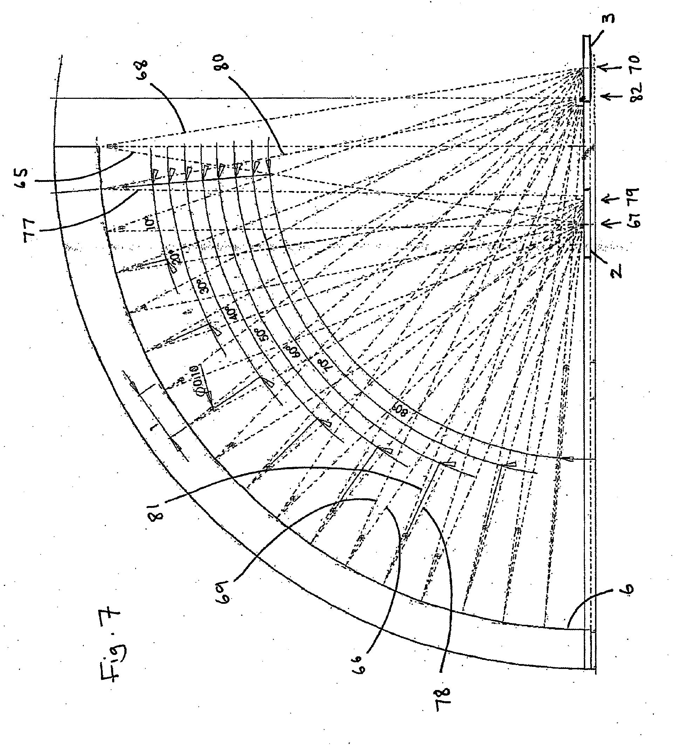

The dome functions to reflect, as near as possible, the radiation from the emitter 2 through a single reflection to the identical mirrored location on the detector 3, irrespective of the radiation exit angle from the emitter. This is illustrated by Figures 6 and 7.

With reference to Figure 6, light rays 65, 66 leaving the centre 67 of the emitter 2 are reflected by the inner surface 6 as rays 68, 69 to the centre of the detector 70. Light rays 71, 72 leaving the outer side 73 of the emitter 2 are reflected as rays 74, 75 to the outer side of the detector 76.

With reference to Figure 7, as for Figure 6, light rays 65, 66 leaving the centre 67 of the emitter 2 are reflected by the inner surface 6 as rays 68, 69 to the centre of the detector 70. Light rays 77, 78 leaving the inner side 79 of the emitter 2 are reflected as rays 80, 81 to the inner side of the detector 82.

The emitter and detector are positioned on a common mounting a small distance apart, typically 3mm centre to centre. The mutually tangent radii forming the swept profile of the toroid are constructed such that the path length from emitter and detector is on average equal. The number of tangent radii used to construct the swept profile of the toroid determines the variation in path length for each ray angle between emitter and detector, therefore the statement that the path length for each specific radii is on average equal refers to the average path length of each radius, which generally occurs at the radius mid point.. Therefore, apart from one specific point in each radius, the path length varies continuously across the face of each radius and therefore the face of the toroidal swept curve, within the limits imposed by the number of radii selected in the construction of the swept curve.

This dome has the effect of transferring the image of the emitter to the detector, without a focus. The image may be concentrated towards the centre of the detector when the components become out of alignment. This increases the manufacturing tolerance of the assembly.

The surface is formed by silver coating an injection moulded feature that forms part of the sensor housing and does not provide a mounting for the emitter and detector.

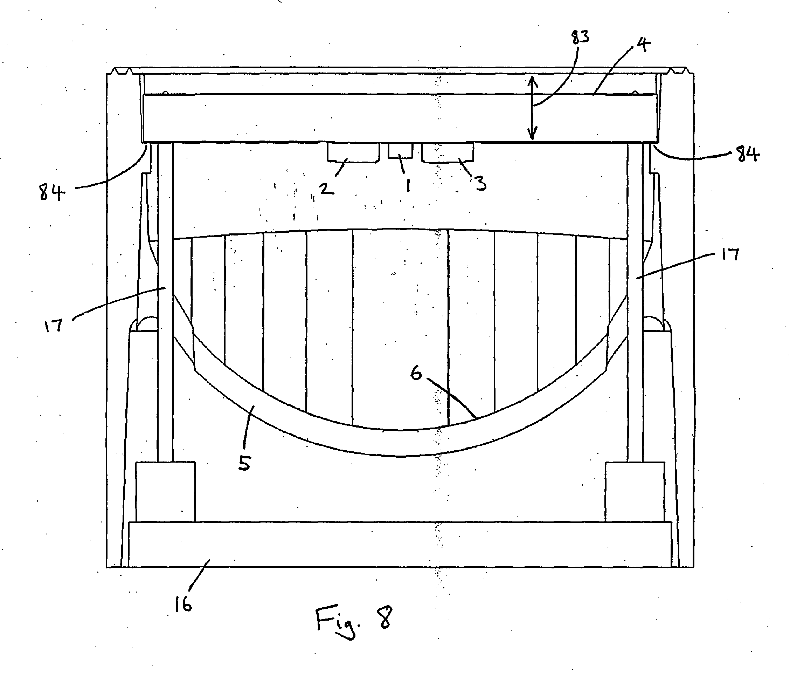

With reference to Figure 8, the emitter 2 and detector 3 are bonded to a bridge PCB (printed circuit board) 4 that serves to provide electrical connectivity, thermal path, and a mounting means that is adjustable on assembly to optimise the location of the reflected radiation pool on the detector. Other components are labelled as in previous figures. A typical surface emitting LED may have an emission surface that is 1 mm² in area, and the location of the bridge mounting the emitter and detector is adjusted to provide as near as possible a pool of radiation (nearly identical to that radiated) striking the detector photodiode (as the non-focussed lighting provides greater efficiency) that is the same size. If the bridge PCB is wrongly adjusted in either direction, at no time will the total emitted radiation focus to a single point. The adjustment is performed when the bridge PCB is raised or lowered in the direction shown 83 in response to feedback, such as the detector received signal strength. When the adjustment is optimum, the location of the bridge 4 with respect to the dome 5 is locked, for example by gluing the PCB interconnect pins into the dome 5. Soldering may also be used, for example by soldering the pins to lock the pins in position in the bridge PCB 4 or the base PCB 16. Other forms of providing the adjustment to an optimum position may be used, for example the adjustment may move the bridge 4 with respect to the pins 17 and the position may be locked by affixing the bridge to the pins after adjustment. Soldering may also be used for locking, for example by soldering the pins in position in the bridge PCB 4 or the base PCB 16. The bridge PCB stop 84 acts as a limit to the adjustment during assembly and prevents the assembly falling apart in case the locking means fails during use.

There is no limitation to the range of angles that light emitted from the LED can reflect within the housing with the exception of the natural optical reflectivity of the emitter and detector surfaces. Therefore typically radiation up to around 80 degree half angle may find its way from the emitter to a similar location on the detector.

In the case where multiple gases are to be detected with the single housing or multiple emitter or detector elements are required, these are grouped as previously described for the single LED and photodiode except that in the case where multiple frequency LEDs are required these will be clustered in an area that is equal to or less than the area of the detector. In this configuration radiation from the multiple emitters will strike the detector in a similar corresponding location to that of emission from the LED. In the case where multiple detectors are required the same principle will apply. Also for any combination of emitter and or detector numbers the same principle will apply.

Further modifications and improvements may be made without departing from the scope of the invention herein described by the claims.

## Claims

1. A gas sensor comprising:
a radiation source (2);
a radiation detector (3); and
a reflecting means (6) arranged to reflect radiation from the radiation source (2) to the
radiation detector (3) along an optical path, wherein the radiation source (2) and the radiation detector (3) are disposed side by side and the reflecting means (6) is arranged to reflect radiation (65, 66, 71, 72, 77, 78) divergent from the radiation source (2) and to concentrate the reflected radiation (68, 69, 74, 75, 80, 81) onto the radiation detector (3), and
wherein the reflecting means (6) comprises a surface comprising a plurality of sub surfaces (51-59), each defined by an arc with a radius and a centre point (60),
**characterised by**
the sub surfaces being swept out by rotation of the arcs around an axis (64), and each sub surface (51-59) being tangent to an adjacent sub surface (51-59) and having a different radius and different centre point (60) from the adjacent sub surface (51-59).

2. The gas sensor of claim 1 wherein the axis (64) is in line with the radiation source (2) and the radiation detector (3).

3. The gas sensor of claim 1 or claim 2 wherein the arc length tends to zero.

4. The gas sensor of any previous claim wherein the sub surfaces (51-59) are semi-toroidal.

5. The gas sensor of any previous claim wherein the surface (6) is configured to reflect radiation from the radiation source (73, 79) to a corresponding location (76, 82) on the radiation detector, irrespective of the radiation exit angle from the radiation source.

6. The gas sensor of any previous claim wherein the surface (6) is configured to reflect radiation leaving the centre of the radiation source (67) to the centre of the radiation detector (70), radiation leaving the outer side of the radiation source (73) to the outer side of the radiation detector (76), and radiation leaving the inner side of the radiation source (79) to the inner side of the radiation detector (82).

7. The gas sensor of any previous claim wherein the surface (6) is configured to reflect radiation such that the length of the optical path is on average equal for each sub surface.

8. The gas sensor of any previous claim wherein the reflecting means (6) comprises a reflective surface of a housing (5).

9. The gas sensor of claim 8 wherein the housing (5) has at least one aperture (13, 14) for permitting the transport of gas in and out of the gas sensor.

10. The gas sensor of any previous claim wherein the radiation source (2) and radiation detector (3) are mounted on a common substrate (4) configured to locate the radiation source (2) and radiation detector (3) in relation to the housing (5).

11. The gas sensor of claim 10 wherein the substrate is configured as an elongate member (4) extending along a diameter of the housing (5).

12. The gas sensor of any previous claim wherein the elongate member (4) is adjustable so as to optimise the location of a reflected radiation pool on the radiation detector (3).

13. The gas sensor of claim 12 wherein the elongate member (4) is adjustable by sliding of pins (17).

14. The gas sensor of claim 13 wherein the pins (17) are electrical leads.

15. The gas sensor of any of claims 13 to 14 wherein the adjustable elongate member (4) is lockable with respect to the reflecting means (6).

16. The gas sensor of claim 15 wherein the adjustable elongate member (4) is lockable by gluing the pins (17) to the reflecting means (6).

17. The gas sensor of claim 15 wherein the adjustable elongate member (4) is lockable by soldering the pins (17).

18. The gas sensor of any previous claim wherein the reflecting means (6) is arranged such that the optical path is defined at least in part by a cavity extending around the radiation source (2) and radiation detector (3).

19. The gas sensor of claim 18 wherein the cavity is bounded by a plane parallel to surfaces of the radiation source (2) and the radiation detector (3).

20. The gas sensor of any previous claim wherein the surface (6) is configured such that radiation originating from a point on the radiation source (2) is unfocussed as it converges on the radiation detector (3).

## Patentansprüche

1. Gassensor, umfassend:
eine Strahlungsquelle (2);
einen Strahlungsdetektor (3); und
ein Reflexionsmittel (6), das zum Reflektieren von Strahlung von der Strahlungsquelle (2) zu dem Strahlungsdetektor (3) entlang einem Strahlenverlauf angeordnet ist, wobei die Strahlungsquelle (2) und der Strahlungsdetektor (3) nebeneinander angeordnet sind und das Reflexionsmittel (6) dazu angeordnet ist, von der Strahlungsquelle (2) divergierende Strahlung (65, 66, 71, 72, 77, 78) zu reflektieren und die reflektierte Strahlung (68, 69, 74, 75, 80, 81) auf den Strahlungsdetektor (3) zu konzentrieren, und
wobei das Reflexionsmittel (6) eine Fläche umfasst, die mehrere Teilflächen (51-59) umfasst, die jeweils durch einen Bogen mit einem Radius und einem Mittelpunkt (60) definiert werden,
**dadurch gekennzeichnet, dass**
die Teilflächen durch Drehung der Bögen um eine Achse (64) überstrichen werden und jede Teilfläche (51-59) tangential zu einer benachbarten Teilfläche (51-59) verläuft und einen anderen Radius und einen anderen Mittelpunkt (60) als die benachbarte Teilfläche (51-59) aufweist.

2. Gassensor nach Anspruch 1, wobei die Achse (64) in einer Linie mit der Strahlungsquelle (2) und dem Strahlungsdetektor (3) verläuft.

3. Gassensor nach Anspruch 1 oder 2, wobei die Bogenlänge gegen null tendiert.

4. Gassensor nach einem vorhergehenden Anspruch, wobei die Teilflächen (51-59) semi-toroidal sind.

5. Gassensor nach einem vorhergehenden Anspruch, wobei die Fläche (6) dazu konfiguriert ist, unabhängig von dem Strahlungsaustrittswinkel von der Strahlungsquelle Strahlung von der Strahlungsquelle (73, 79) zu einer entsprechenden Stelle (76, 82) auf dem Strahlungsdetektor zu reflektieren.

6. Gassensor nach einem vorhergehenden Anspruch, wobei die Fläche (6) dazu konfiguriert ist, die Mitte der Strahlungsquelle (67) verlassende Strahlung zu der Mitte des Strahlungsdetektors (70) zu reflektieren, die äußere Seite der Strahlungsquelle (73) verlassende Strahlung zu der äußeren Seite des Strahlungsdetektors (76) zu reflektieren und die innere Seite der Strahlungsquelle (79) verlassende Strahlung zu der inneren Seite des Strahlungsdetektors (82) zu reflektieren.

7. Gassensor nach einem vorhergehenden Anspruch, wobei die Fläche (6) dazu konfiguriert ist, Strahlung so zu reflektieren, dass die Länge des Strahlenverlaufs im Durchschnitt für jede Teilfläche gleich ist.

8. Gassensor nach einem vorhergehenden Anspruch, wobei das Reflexionsmittel (6) eine reflektierende Fläche eines Gehäuses (5) ist.

9. Gassensor nach Anspruch 8, wobei das Gehäuse (5) mindestens eine Öffnung (13, 14) aufweist, um den Transport von Gas in den und aus dem Gassensor zu gestatten.

10. Gassensor nach einem vorhergehenden Anspruch, wobei die Strahlungsquelle (2) und der Strahlungsdetektor (3) auf einem gemeinsamen Träger (4) montiert sind, der dazu konfiguriert ist, die Strahlungsquelle (2) und den Strahlungsdetektor (3) bezüglich des Gehäuses (5) zu positionieren.

11. Gassensor nach Anspruch 10, wobei der Träger als ein längliches Glied (4) konfiguriert ist, das sich entlang einem Durchmesser des Gehäuses (5) erstreckt.

12. Gassensor nach einem vorhergehenden Anspruch, wobei das längliche Glied (4) dazu einstellbar ist, die Position eines reflektieren Strahlungspools auf dem Strahlungsdetektor zu optimieren.

13. Gassensor nach Anspruch 12, wobei das längliche Glied (4) durch Verschieben von Stiften (17) einstellbar ist.

14. Gassensor nach Anspruch 13, wobei die Stifte (17) elektrische Leitungen sind.

15. Gassensor nach einem der Ansprüche 13 bis 14, wobei das einstellbare längliche Glied (4) bezüglich des Reflexionsmittels (6) verriegelbar ist.

16. Gassensor nach Anspruch 15, wobei das einstellbare längliche Glied (4) durch Verkleben der Stifte (17) mit dem Reflexionsmittel (6) verriegelbar ist.

17. Gassensor nach Anspruch 15, wobei das einstellbare längliche Glied (4) durch Verlöten der Stifte (17) verriegelbar ist.

18. Gassensor nach einem vorhergehenden Anspruch, wobei das Reflexionsmittel (6) so angeordnet ist, dass der Strahlenverlauf zumindest teilweise durch einen sich um die Strahlungsquelle (2) und den Strahlungsdetektor (3) erstreckenden Hohlraum definiert wird.

19. Gassensor nach Anspruch 18, wobei der Hohlraum durch eine parallel zu den Flächen der Strahlungsquelle (2) und des Strahlungsdetektors (3) verlaufende Ebene begrenzt wird.

20. Gassensor nach einem vorhergehenden Anspruch, wobei die Fläche (6) dazu konfiguriert ist, dass von einem Punkt auf der Strahlungsquelle (2) ausgehende Strahlung nicht fokussiert wird, wenn sie auf dem Strahlungsdetektor (3) konvergiert.

## Revendications

1. Capteur de gaz comprenant :
une source de rayonnement (2) ;
un détecteur de rayonnement (3) ; et
des moyens de réflexion (6) disposés pour réfléchir le rayonnement provenant de la source de rayonnement (2) vers le détecteur de rayonnement (3) le long d'un trajet optique,
la source de rayonnement (2) et le détecteur de rayonnement (3) étant disposés côte à côte et les moyens de réflexion (6) étant disposés pour réfléchir le rayonnement (65, 66, 71, 72, 77, 78) de manière divergente de la source de rayonnement (2) et pour concentrer le rayonnement réfléchi (68, 69, 74, 75, 80, 81) sur le détecteur de rayonnement (3), et
les moyens de réflexion (6) comprenant une surface comprenant une pluralité de surfaces secondaires (51-59), chacune définie par un arc ayant un rayon et un centre (60),
**caractérisé en ce que**
les surfaces secondaires sont étalées par rotation des arcs autour d'un axe (64) et
chaque surface secondaire (51-59) est tangente à une surface secondaire (51-59) voisine et possède un rayon et un centre (60) différents de la surface secondaire (51-59) voisine.

2. Capteur de gaz selon la revendication 1, l'axe (64) étant aligné avec la source de rayonnement (2) et le détecteur de rayonnement (3).

3. Capteur de gaz selon la revendication 1 ou la revendication 2, la longueur de l'arc tendant vers zéro.

4. Capteur de gaz selon l'une quelconque des revendications précédentes, les surfaces secondaires (51-59) étant semi-toroïdales.

5. Capteur de gaz selon l'une quelconque des revendications précédentes, la surface (6) étant configurée pour réfléchir le rayonnement provenant de la source de rayonnement (73, 79) vers un emplacement (76, 82) correspondant sur le détecteur de rayonnement, indépendamment de l'angle de sortie du rayonnement depuis la source de rayonnement.

6. Capteur de gaz selon l'une quelconque des revendications précédentes, la surface (6) étant configurée pour réfléchir le rayonnement qui quitte le centre de la source de rayonnement (67) vers le centre du détecteur de rayonnement (70), le rayonnement qui quitte le côté extérieur de la source de rayonnement (73) vers le côté extérieur du détecteur de rayonnement (76) et le rayonnement qui quitte le côté intérieur de la source de rayonnement (79) vers le côté intérieur du détecteur de rayonnement (82).

7. Capteur de gaz selon l'une quelconque des revendications précédentes, la surface (6) étant configurée pour réfléchir le rayonnement de telle sorte que la longueur du trajet optique est en moyenne égale pour chaque surface secondaire.

8. Capteur de gaz selon l'une quelconque des revendications précédentes, les moyens de réflexion (6) comprenant une surface réfléchissante d'un boîtier (5).

9. Capteur de gaz selon la revendication 8, le boîtier (5) comprenant au moins une ouverture (13, 14) pour permettre le transport de gaz vers l'intérieur et l'extérieur du capteur de gaz.

10. Capteur de gaz selon l'une quelconque des revendications précédentes, la source de rayonnement (2) et le détecteur de rayonnement (3) étant montés sur un substrat commun (4) configuré pour positionner la source de rayonnement (2) et le détecteur de rayonnement (3) par rapport au boîtier (5).

11. Capteur de gaz selon la revendication 10, le substrat étant configuré sous la forme d'un élément allongé (4) qui s'étend le long d'un diamètre du boîtier (5).

12. Capteur de gaz selon l'une quelconque des revendications précédentes, l'élément allongé (4) étant réglable de manière à optimiser l'emplacement d'un concentré de rayonnement réfléchi sur le détecteur de rayonnement (3).

13. Capteur de gaz selon la revendication 12, l'élément allongé (4) étant réglable en faisant coulisser des broches (17).

14. Capteur de gaz selon la revendication 13, les broches (17) étant des fils électriques.

15. Capteur de gaz selon l'une quelconque des revendications 13 et 14, l'élément allongé (4) réglable pouvant être verrouillé par rapport aux moyens de réflexion (6).

16. Capteur de gaz selon la revendication 15, l'élément allongé (4) réglable pouvant être verrouillé en collant les broches (17) aux moyens de réflexion (6).

17. Capteur de gaz selon la revendication 15, l'élément allongé (4) réglable pouvant être verrouillé en soudant les broches (17).

18. Capteur de gaz selon l'une quelconque des revendications précédentes, les moyens de réflexion (6) étant disposés de telle sorte que le trajet optique est défini au moins en partie par une cavité qui s'étend autour de la source de rayonnement (2) et du détecteur de rayonnement (3).

19. Capteur de gaz selon la revendication 18, la cavité étant délimitée par un plan parallèle aux surfaces de la source de rayonnement (2) et du détecteur de rayonnement (3).

20. Capteur de gaz selon l'une quelconque des revendications précédentes, la surface (6) étant configurée de telle sorte que le rayonnement qui émane d'un point de la source de rayonnement (2) n'est pas concentré lorsqu'il converge sur le détecteur de rayonnement (3).
